# EUROPEAN PATENT APPLICATION

(11) **EP 2 740 488 A1**
(43) Date of publication of application: **11.06.2014**
(21) Application number: 11869551.9
(22) Date of filing: 23.12.2011
(51) Int. Cl.: A61K 39/21, A61P 31/18

(54) **SEQUENTIAL AND REPEATED APPLICATION OF FOUR OR MORE HIV VECTOR GENE VACCINES**

(30) Priority: 18.07.2011 CN 201110200297
(71) Applicant: Zeng, Yi, Beijing 100052 (CN); Feng, Xia, Beijing 100052 (CN); Yu, Shuangqing, Beijing 100052 (CN); Yang, Ling, Beijing 100052 (CN); Li, Zelin, Beijing 100124 (CN); Kong, Wei, Changchun, Jilin 130012 (CN); Yu, Xianghui, Changchun, Jilin 130012 (CN)
(72) Inventor: Zeng, Yi, Beijing 100052 (CN); Feng, Xia, Beijing 100052 (CN); Yu, Shuangqing, Beijing 100052 (CN); Yang, Ling, Beijing 100052 (CN); Li, Zelin, Beijing 100124 (CN); Kong, Wei, Changchun, Jilin 130012 (CN); Yu, Xianghui, Changchun, Jilin 130012 (CN)
(74) Representative: Tetzner, Michael
(86) International application number: PCT/CN2011/002174
(87) International publication number: WO 2013/010300

(57) **Abstract**

The present invention provides a combined AIDS vaccine for preventing and/or treating AIDS, which consists of four or more different vector-based AIDS vaccines, wherein HIV protein gene comprised in the different AIDS vaccines may be same. A use method of the vaccine comprises inoculation of one AIDS vaccine each time, wherein inoculation of each vaccine may be continuously performed twice, and after sequential application of the four or more vector vaccines, repeated sequential application of the vector vaccines may be performed.

## Description

### TECHNICAL FIELD

The invention belongs to the biomedical field. The present invention relates to the immunization strategy of sequential and repeated application of four or more vector-based HIV gene vaccines. The vaccines to be used in any particular application will not be limited to the four vaccines used in the present preliminary study.

### BACKGROUND

Human immunodeficiency virus (HIV) belongs to the human lentivirus group of lentivirus genus of Retroviridae family, is the pathogen causing the acquired immunodeficiency syndrome, i.e. AIDS, and was identified for the first time in 1983. AIDS is considered to be the first major infectious disease in the world which directly threats the human health and has become a major public health problem worldwide. The 2009 report on the global AIDS epidemic jointly issued by the Joint United Nations Programme on HIV and AIDS (UNAIDS) and the World Health Organization (WHO) shows that, there were about 33.4 million people living with HIV in the world, and in 2008 there were 2.7 million new infections and 200 million people died of AIDS-related diseases. Since 1983 when the first case of HIV infection occurred in China, as of the end of October 2010, there reported a total of 370, 000 cases of HIV infections and patients, with more than 130, 000 cases of patient and 6.8 million cases of death.

AIDS vaccine is an important means to control AIDS. For a long time scientists wish to develop a vaccine to prevent HIV infection. It was considered early in the AIDS epidemic that neutralizing antibodies are sufficient to prevent HIV infection. However, earlier experiments suggested that the antibodies produced by the monomeric HIV envelope protein cannot prevent and control HIV infection, mainly due to the inability of immunogens to produce broadly neutralizing antibodies. Therefore, the focus was shifted to inducing cellular immune responses from inducing neutralizing antibodies. The research carried out on this basis suggested that the specific cytotoxic T cell responses are related to a decrease of viral load, but no significant protective effect of prophylactic vaccines for inducing cellular immune was observed in the clinical trial. The failure of such vaccines may be related to the following factors: the vaccine immunization can produce relatively high but short-duration specific cellular immune responses which, even if partly effective, cannot prevent HIV infection. As the progress in understanding of AIDS immune protection mechanism is slow and the development of the vaccine for completely preventing HIV-1 infection has yet to be a breakthrough, it is very important to correctly understand and evaluate the vaccine's effect. More and more studies have shown that lowering the viral load in HIV infected persons can delay the progression to AIDS, reduce the possibility of transmission to others, thus limiting the prevalence of HIV-1. As HIV specific immune responses have been produced in HIV infected persons, the vaccine immunization to enhance the immune responses to the specific antigen, in theory, can play a certain role in controlling virus replication, reducing viral load and slowing disease progression. At present, more than 50 clinical trials of HIV therapeutic vaccines have been completed or ongoing, most of these vaccines take single application of one vaccine or take co-application of two vaccines in prime-boost scheme. It is very important in design of therapeutic vaccines to take into consideration the repeated application of the vaccine, because these patients may need lifelong treatment. Although vector vaccines can induce good cellular immune responses, the immunogenicity of vector itself greatly limits the repeated application of the vaccine. Therefore, it is necessary to sequentially apply more than one vector vaccines which will induce HIV-specific immune responses, such that the effect of more than one treatment in a patient can be achieved. When the specific immune responses induced by one vaccine immunization drop to a certain level, giving another vaccine immunization can strengthen the existing specific immune responses. Similarly, when the specific immune responses drop again, the third and the fourth vaccine immunization will be performed sequentially. By such sequential immunization of four vaccines, a higher level of immune responses in a recipient may be maintained for a long period. After a round of sequential immunization of four vaccines is completed, a second round can be performed to repeat the application. In this way, the specific immune responses in a recipient can be maintained for a longer period. If necessary, a new vector vaccine can be added. We have experimentally verified this design idea.

### SUMMARY OF THE INVENTION

The objective of the present invention is to provide a strategy of sequential and repeated application of four or more vector vaccines containing multiple HIV genes, which can maintain high level of specific immune responses for a long period.

The technical solution of the combined vaccine of the present invention is as follows: a combined vaccine against HIV, which can activate broad spectrum of anti-HIV cellular immune responses and consists of four or more AIDS vaccines, wherein the HIV genes carried in the different AIDS vaccines can be same or different, each AIDS vaccine is used alone to perform at least one inoculation, that is, at least four, in total, inoculations are performed.

The AIDS vaccines can be DNA vaccines encoding HIV envelope proteins or core proteins, recombinant viral vector vaccines, recombinant bacterial vector vaccines, recombinant yeast vector vaccines, or recombinant virus-like particle vaccines, or the like.

The genes encoding HIV envelope proteins can at least be gp120, gp140, gp145, gp160, or encoding genes containing above gene fragments; the core protein genes is full-length or partial fragments of gag coding sequence..

The HIV envelope protein or core protein genes can be derived from HIV-1 or HIV-2, or from different subtypes or strains of HIV-1, or can also be derived from different forms of same strain, for example (but not limited to) gp140 and gp145 derived from same subtype are considered to be different envelope proteins or genes The sequential and repeated immunization of the different AIDS vaccines is performed to activate cellular immune responses against the virus.

The technical solution of the sequential and repeated application of the combined multi-vector gene vaccine of the present invention is as follows: a method of combining vaccines against various HIVs, performing sequential immunization of four or more AIDS vaccines to activate cellular immune responses against the virus, successively improving the cellular immune responses against the conserved epitopes, and thereby enhancing overall immune responses; in which, HIV envelope protein or core protein genes contained in said different AIDS vaccines can be same. One inoculation is performed with one AIDS vaccine, and the inoculation of each vaccine can be performed twice successively. After sequential application of four or more different vector vaccines is completed, the sequential application can be repeated with the above vector vaccines.

The AIDS vaccines can be DNA vaccines encoding HIV envelope proteins or core proteins, various recombinant viral vector vaccines, recombinant bacterial vector vaccines, recombinant yeast vector vaccines, or recombinant virus-like particle vaccines, etc.

The genes encoding HIV envelope proteins can at least be gp120, gp140, gp145, gp160, or coding genes containing above gene fragments; the core protein genes is full-length or partial fragments of gag coding sequence.

The HIV envelope protein or core protein genes can be derived from HIV-1 or HIV-2, or from different subtypes or strains of HIV-1, or can also be derived from different form of same strain, for example (but not limited to) gp140 and gp145 derived from same subtype are considered to be different envelope proteins or genes

In a particular example of the present invention, we used the following strategy: using plasmid DNA, recombinant adenovirus and recombinant Sendai virus vaccines which express *gag* gene of B' subtype, and recombinant poxvirus vaccines which express *gag-pol* and *env* gene of recombinant B'/C subtype, to perform sequential and repeated application. Rhesus macaques were immunized primarily twice with a DNA vaccine, then boosted twice with a recombinant adenovirus or recombinant Sendai virus vaccine as the second or third vaccine, and lastly immunized twice with a recombinant poxvirus vaccine. The ELISPOT method was used to detect cellular immune responses and it was found that the Rhesus macaques inoculated with four vaccines can maintain high level of specific immune responses for a long period.

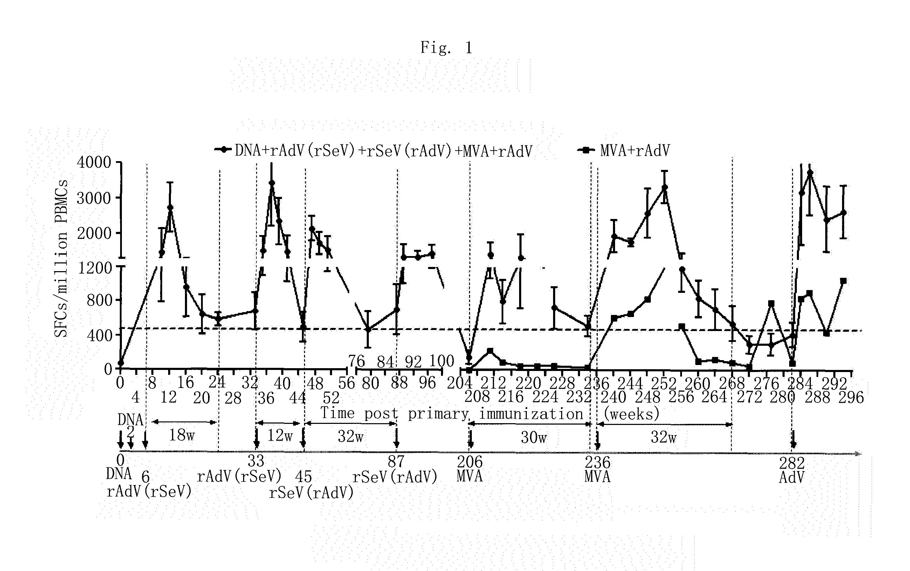

A second round of vaccine immunization was performed after the end of a round of immunization of four vaccines, the result showing that just the first immunization with recombinant adenovirus vaccine in the second round can maintain high level of specific immune responses for a longer period. (See Fig. 1)

The advantages of the present invention are, for example, as follows:

The fourth vaccine is added sequentially into our earlier sequential application of three vaccines and expresses antigens different from those of the earlier three vaccines, the result showing that high level of subtype cross-reactive immune responses can be well induced and maintained for an extended period.

The sequential and repeated application of four vaccines has not been reported in any literature, and can stimulate potent specific immune responses which can be maintained at high level for a long period in our study.

### BRIEF DESCRIPTION OF THE DRAWINGS

Figure 1 is a graph showing the specific cellular immune responses in Rhesus macaques immunized with four vaccines sequentially and repeatedly, as detected at different time points by ELISPOT method.

### DETAILED DESCRIPTION OF THE EMBODIMENTS

### The experiment for immunizing Rhesus macaques with HIV vaccines sequentially and repeatedly

### 1. Vaccine immunization and grouping

8 adult rhesus macaques were randomly divided into three groups, in which: the first group of 2 rhesus macaques is PBS control group, receiving intramuscular injection of 1 ml sterile PBS at the same time points as the experiment group; the second group of 5 rhesus macaques is four-vaccines immunization group, immunized primarily with a DNA vaccine (the DNA vaccine used is an eukaryotic expression plasmid encoding codon-optimized gag gene, which is pVR-mod.gag as constructed in the prior patent application No. 200610098946.1 filed in 2006 by the present applicant and manufactured by Vector Gene Technology Company Ltd.) twice at week 0 and week 2 respectively, then immunized with same dosage of adenovirus vaccine rAd5-gag (rAd5-gag is replication-defective recombinant adenovirus type 5 expressing mod.gag gene with E1 and E3 deletion, mCMV promoter and polyadenylation signal of SV40 polyA, which is rAd5-mod.gag as constructed in the prior patent application No. 200710000534.4 filed in 2007 by the present applicant and manufactured by Vector Gene Technology Company Ltd.) or Sendai virus vaccine rSeV-gag (rSeV-gag is recombinant F gene-defective Sendai virus vector vaccine expressing wt.gag269 genes, provided by DNAVEC Corporation) at week 6 and week 33, boosted with Sendai virus vaccines (adenovirus vaccines) (*same as above*) at week 45 and week 87, and immunized with recombinant poxvirus vaccine rMVA (rMVA is recombinant MVA vaccine expressing B'/C recombinant subtype HIV *gag-pol* and *env* genes, provided by Changchun BCHT Biotechnology Co.) at week 206 and week 236, receiving the second round of immunization at week 282 with adenovirus vaccine firstly. The third group of 1 rhesus macaque is two-vaccines immunization group, receiving intramuscular injection of 1 ml sterile PBS at weeks 0, 2, 6, 33, 45 and 87, immunized with recombinant poxvirus vaccine rMVA (*same as above*) at week 206 and week 236, and immunized with adenovirus vaccine rAd5-gag (rAd5-gag is replication-defective recombinant adenovirus type 5 expressing mod.gag gene with E1 and E3 deletion, mCMV promoter and polyadenylation signal of SV40 polyA, which is rAd5-mod.gag as constructed in the prior patent application No. 200710000534.4 filed in 2007 by the present applicant and manufactured by Shenzhen Tsinghua Yuanxing Bio-Pharm Co., Ltd.) at week 282. The dosage of each vaccine used is respectively as follows: DNA 5 mg/monkey, rAd5-gag 10⁹ PFU/monkey, rSeV-gag 10⁹ CIU/monkey, rMVA 9×10⁷ PFU/monkey. See Table 1 for the detail of the grouping and immunization.

**Table 1: The Grouping and Immunization of Rhesus Macaques**

| Grouping | Nos. of Monkeys | Time Points of Immunization and vaccines | | | | |
|---|---|---|---|---|---|---|
| | | 0w,2w | 6w,33w | 45w,87w | 206w,236w | 282w |
| PBS Control | 98R0009 | PBS | PBS | PBS | PBS | PBS |
| | 00R0023 | PBS | PBS | PBS | PBS | PBS |
| Four-vaccines Immunization Group | 98R0053 | DNA | rAd5-gag | rSeV-gag | rMVA | rAd5-gag |
| | 00R0019 | DNA | rAd5-gag | rSeV-gag | rMVA | rAd5-gag |
| | 98R0013 | DNA | rSeV-gag | rAd5-gag | rMVA | rAd5-gag |
| | 00R0227 | DNA | rSeV-gag | rAd5-gag | rMVA | rAd5-gag |
| | 99R0035 | DNA | rSeV-gag | rAd5-gag | rMVA | rAd5-gag |
| Two-Vaccines Immunization Group | 98R0037 | PBS | PBS | PBS | rMVA | rAd5-gag |

### 2. Detection of the level of Gag specific cellular immune responses in immunized Rhesus macaques by ELISPOT method

ELISPOT technique is so far the most sensitive and widely used technique for ex vivo monitoring of antigen-specific T cells. Specific cellular immunity refers to that the T cells, after stimulated by an foreign antigen, proliferate, differentiate, and transform into sensitized T cells which can directly lyse target cells expressing foreign antigen or release lymphokines to synergistically kill target cells when the same antigen enter the body again, thereby clear the infected cells in the body. In an ELISPOT experiment, the level of cellular immune responses is evaluated by enumerating cytokine(such as IFN-γ)-secreting lymphocytes after stimulated with specific stimulus in vitro; The specific stimulus may be polypeptides, gene expression products and extracted antigens, etc, which can mimic the in vivo antigen stimulation. EDTA anticoagulant peripheral venous blood was sampled in 10ml/monkey at different time points after immunization, and within 8 hours after the blood sampling, peripheral blood lymphocytes were separated using monkey lymphocyte separation solution (EZ-Sep™ Monkey 9 X, Shenzhen Dakewe Biotech Co., Ltd., Catalog No. DKW33-H0100). The number of lymphocytes secreting IFN-γ after stimulated by Gag specific peptide pool was determined by Enzyme-linked Immunospot Assay (ELISPOT). Gag269 peptide pool is from University of Tokyo, Japan (gag269 gene was separated from the blood sample of a HIV-1-positive people in 1990s by Dr. Guan Yongjun in our laboratory, and the peptide pool derived from the amino acid sequence of that gene was synthesized by our cooperator, University of Tokyo, Japan (Yu S, Feng X, Shu T, Matano T, Hasegawa M, Wang X, Ma H, Li H, Li Z, Zeng Y: Potent specific immune responses induced by prime-boost-boost strategies based on DNA, adenovirus, and Sendai virus vectors expressing gag gene of Chinese HIV-1 subtype B. Vaccine 2008, 26:6124-6131.), consists of 124 short peptides in length of 14-16aa, in which two adjacent peptides have 11aa overlap and each peptide has a purity of 52%-100%, and the stock solution is dissolved in DMSO. Gag269 peptide pool was divided, according to the order from N terminal to C terminal of amino acid sequences, into five peptide sub-pools, respectively named as AB (aal-105), CD (aa95-203), EF (aa193-305), GH (aa295-408) and IJ (aa398-516). These peptide sub-pools were adjusted to 2µM concentration and were added successively into 96-well ELISPOT plate coated with IFN-γ capture antibodies, each sub-pool in duplicate wells in 50µl/well and a negative control well (1 % DMSO for co-culture) and a positive control well (in final concentration, 50ng/ml PMA (Sigma, Germany, Catalog No. p1585) and 1µg/ml of Ionomycin (Sigma, Germany, Catalog No. I9657)) for each sample, and then 50µl PBMC in 4×10⁶/ml concentration was added into each well. After all of samples were added, the plate was covered, placed into Carbon dioxide incubator, and incubated for 36h at 37°C in 5% CO₂. After incubation, the plate was reacted respectively with the biotin labeled primary antibody and the HRP labeled Streptavidin, and lastly was developed with AEC developing solution.

The detection result is shown in Fig 1 which however does not show the result of control group since the immune responses at all detection points in the control group are negative. In the four-vaccine immunization group very low level of immune responses were induced after DNA vaccination, but the responses peaked at 2744SFCs/10⁶PMBCs very soon after immunization with the first viral vector vaccine and maintained above 500SFCs/10⁶PMBCs for 18 weeks, and again the induced immune responses peaked at 3424 SFCs/10⁶PMBCs very soon after the second immunization and maintained above 500SFCs/10⁶PMBCs for 12 weeks. Also very soon after one immunization with the second viral vector vaccine, the induced immune responses reached a peak value lower than that induced by the first viral vector vaccine but maintained at the level above 500SFCs/10⁶PMBCs for a significant longer period of 34 weeks. The first and second immunization with the third viral vector vaccine also induced respectively a peak of immune responses, even up to 3339SFCs/10⁶PMBCs after the second immunization, and maintained the level above 500SFCs/10⁶PMBCs for 30 weeks and 32 weeks respectively. When starting the immunization with the fourth vaccine, a Rhesus macaque with earlier PBS inoculation was also immunized with the rMVA vaccine, and produced very weak responses after the first immunization and relative stronger responses after the second immunization which, however, was far lower than that of the four-vaccine immunization group and dropped to a lower level very soon. In the four-vaccine immunization group, after the immunization with the first viral vector vaccine and before week 266 (about 5 years), the average level of cellular responses was always above 500SFCs/10⁶ PMBCs, and just 8 co-immunizations with four vaccines can maintain a certain level of cellular responses in an inoculated subject for a long period. From week 282, the four-vaccine immunization was repeated for which the adenovirus vaccine was selected as the first vaccine, and the immune responses in the four-vaccine group peaked very soon after inoculation and was monitored still at a very high level at week 14 after immunization. Comparatively, in the Rhesus macaque with earlier MVA inoculation, the responses after inoculated with the adenovirus vaccine were significant lower than that of the four-vaccine group.

Above experiments for immunizing Rhesus macaques with multi-vector based four vaccines suggest that the sequential and repeated application of four vaccines can induce specific immune responses many times and for a longer period.

Although the invention has been sufficiently described in connection with specific preferred embodiments, it should be understood that the invention as claimed can have a variety of transformation and modification and should not be unduly limited to such specific embodiments. The above embodiments are only for illustrating but not limiting the invention. Indeed, the protection scope of the present invention should encompass all of transformation, substitution and modification apparent to those of ordinary skill in the art.

## Claims

1. A combined AIDS vaccine for preventing and/or treating AIDS, **characterized in that**, the combined AIDS vaccine can activate broad spectrum of anti-HIV cellular immune responses, the combined AIDS vaccine consists of four or more vector based AIDS vaccines, wherein the HIV protein genes comprised in the different AIDS vaccines can be same, one inoculation is performed with one AIDS vaccine and the inoculation of each vaccine can be performed twice successively, and after sequential application of the four or more different vector vaccines is completed, the sequential application can be repeated with the above vector vaccines.

2. The combined vaccine against various HIVs according to claim 1, **characterized in that**, said AIDS vaccines can be DNA vaccines containing genes encoding HIV envelope proteins or core proteins, recombinant viral vector vaccines, recombinant bacterial vector vaccines, recombinant yeast vector vaccines, or recombinant virus-like particle vaccines.

3. The combined vaccine against various HIVs according to claim 1, **characterized in that**, said genes encoding HIV envelope proteins can at least be gp120, gp140, gp145, gp160, or encoding genes containing above gene fragments; said core protein genes is full-length or partial fragments of gag coding sequence.

4. The combined vaccine against various HIVs according to claim 1, **characterized in that**, said HIV envelope protein or core protein genes can be derived from HIV-1 or HIV-2, or from different subtypes or strains of HIV-1, or can also be derived from different gene types of same strain, for example (but not limited to) gp140 and gp145 derived from same subtype are considered to be different envelope proteins or genes.

5. The combined vaccine against various HIVs according to claim 1, **characterized in that**, the sequential immunization of said different AIDS vaccines in ordered combination is performed to activate cellular immune responses against the conserved region of the virus.

6. A method of using a combined vaccine against various HIVs, **characterized in that**, performing sequential immunization of four or more AIDS vaccines to activate cellular immune responses against the virus, successively improving the cellular immune response against the virus, and thereby enhancing overall immune responses; in which, HIV envelope protein or core protein genes contained in said different AIDS vaccines can be same, one inoculation is performed with one AIDS vaccine, and the inoculation of each vaccine can be performed twice successively, and after completing sequential application of four or more different vector vaccines, the sequential application can be repeated with the above vector vaccines.

7. The method of using a combined vaccine against various HIVs according to claim 6, **characterized in that**, said AIDS vaccines can be DNA vaccines encoding HIV envelope proteins or core proteins, various recombinant viral vector vaccines, recombinant bacterial vector vaccines, recombinant yeast vector vaccines, or recombinant virus-like particle vaccines, or the like.

8. The method of using a combined vaccine against various HIVs according to claim 6, **characterized in that**, said genes encoding HIV envelope proteins can at least be gp120, gp140, gp145, gp160, or coding genes containing above gene fragments; said core protein genes is full-length or partial fragments of gag coding sequence

9. The method of using a combined vaccine against various HIVs according to claim 6, **characterized in that**, said HIV envelope protein or core protein genes can be derived from HIV-1 or HIV-2, or from different subtypes or strains of HIV-1, or can also be derived from different form of same strain.

10. The method of using a combined vaccine against various HIVs according to claim 6, **characterized in that**, performing the sequential and repeated immunization of said different AIDS vaccines to activate cellular immune responses against the virus.
